# EUROPEAN PATENT APPLICATION

(11) **EP 4 714 913 A1**
(43) Date of publication of application: **25.03.2026**
(21) Application number: 25203848.4
(22) Date of filing: 23.09.2025
(51) Int. Cl.: C02F 3/32, C02F 3/00, C02F 3/12

(54) **PROTECTION OF ROTIFERS LECANE AGAINST THE ACTION OF PREDATORY FUNGI ZOOPHAGUS OR LECOPHAGES**

(30) Priority: 23.09.2024 PL 44987724
(71) Applicant: UNIWERSYTET JAGIELLONSKI, 31-007 Kraków (PL)
(72) Inventor: Pajdak - Stós, Agnieszka, 30-065 Kraków (PL); Fialkowska, Edyta, 31-457 Kraków (PL); Korzh, Yuliia, 32-064 Rudawa (PL)
(74) Representative: Witek, Rafal

(57) **Abstract**

Disclosed is a method for limiting the activity of predatory fungi *Zoophagus* or *Lecophagus* that threaten Lecane rotifers capable of reducing the bulking of activated sludge in wastewater treatment plants, which involves the use of a glycerol solution in a concentration ranging from 0.005% to 0.1% in the aeration chamber of an activated sludge system, deactivating the predatory fungi without harming the rotifers.

## Description

The subject of the invention is a method for limiting the activity of predatory fungi that threaten rotifers capable of reducing the bulking of activated sludge in wastewater treatment plants.

These rotifers have proven effective in reducing the number of filamentous bacteria, which cause activated sludge bulking in wastewater treatment plants.

Sludge bulking caused by excessive growth of filamentous bacteria is one of the most common and serious operational problems in wastewater treatment plants around the world. Research to date indicates that rotifers can be used as a biological tool in the fight against filamentous bacteria, competitive against the chemical methods currently in use.

Polish patent application P.383707 describes a method of limiting the growth of filamentous bacteria in activated sludge, characterised in that rotifers and/or testate amoebae are introduced into activated sludge containing filamentous bacteria, and that they ingest the filamentous bacteria and reduce their density in the activated sludge.

Polish patent application P.403846 describes a method for the mass culture of rotifers of the genus *Lecane,* used in particular to reduce the bulking of activated sludge in wastewater treatment plants, applied outside of activated sludge chambers, characterised in that the culture is carried out on dishes, in a liquid medium and with artificial nutrient supplementation, where spring water is used as the medium and a mixture of chicken egg yolk and cocoa is used as the nutrient source.

Rotifers are very important activated sludge organisms because they can increase flocculation, "improve the quality of treated wastewater and control activated sludge bulking caused by filamentous bacteria overgrowth. Unfortunately, their populations are threatened by predatory fungi, which have been found to be surprisingly common in wastewater treatment plants.

Edyta Fiatkowska et al. described in Environmental Science and Pollution Research (2022) 29:17671-17681, https://doi.org/10.1007/s11356-021-16952-2 attempts to protect rotifers from these predatory fungi *Zoophagus* and *Lecophagus,* which involved the use of polyaluminium chloride (PAX-18).

Considering the risk of uncontrolled growth of predatory fungi in wastewater treatment plants, the inventors attempted to find a way to limit their growth. In preliminary studies, an attempt was made to simultaneously apply *Lecane* rotifers, which eat filamentous bacteria, and polyaluminium chloride (PAX-18), commonly used to prevent bulking caused by *M. parvicella,* to activated sludge infected with predatory fungi. The results were unsatisfactory because PAX-18 proved to be harmful not only to the fungi, but also to the rotifers.

The aim of the invention is to protect *Lecane* rotifers from the negative influence of the predatory fungi *Zoophagus* and *Lecophagus.*

The specific aim of the invention is to propose a method suitable for controlling predatory fungi that pose a threat to rotifers used in wastewater treatment plants.

The subject of the invention is the use of glycerol to protect Lecane rotifers against the predatory fungi *Zoophagus* and/or *Lecophagus.*

Preferably, glycerol is used in a concentration ranging from 0.005% to 0.1%.

Preferably, Lecane rotifers are present in the activated sludge.

Another subject of the invention is a method for reducing the bulking of activated sludge that involves the use of rotifers, characterised in that the rotifers are introduced into the activated sludge in the presence of a glycerol solution at a concentration ranging from 0.005% to 0.1%.

In a preferred embodiment, glycerol should be dosed into the treatment plant so that its concentration in the aeration chamber is between 0.005% and 0.1% in the presence of *Zoophagus* fungus and between 0.05% and 0.1% in the presence of *Lecophagus* fungus, with a more pronounced protective effect visible at higher concentrations.

The results of the research that led to the invention unexpectedly showed a strong effect of the addition of glycerol solution on the growth rate of the predatory fungus *Zoophagus sp.,* which was verified experimentally. The experiments were conducted on two 12-well tissue culture plates. Three to five conidia were transferred to each well using a micropipette and then measured. The wells were divided into four groups: Z+R (control); Z+R+GI (0.01%); Z+R+GI (0.05%); Z+R+GI (0.1%), where Z *- Zoophagus sp.,* R - rotifers *L. inermis* and GI - glycerol solution. Thirty µL of a dense culture of rotifers containing approximately 400 individuals was added to each well. Each treatment was performed in six replicates. The experimental plates were incubated in the dark at 20±1°C. The length of the mycelium and all conidia visible in the wells was measured every 24 hours for two consecutive days based on photographs taken using an Olympus IX71 inverted microscope equipped with a Pixellink camera and NIS image analysis system. The measurements were performed using ImageJ software. The growth rate of *Zoophagus* was calculated using the formula *r = 1*/*t (In(Nₜ)-In(Nₜ₀)),* where *t is* the time (days) between measurements, *Nₜ* is the length of the mycelium on the second day of the experiment, and *Nₜ₀* is the length of the mycelium at the beginning of the experiment.

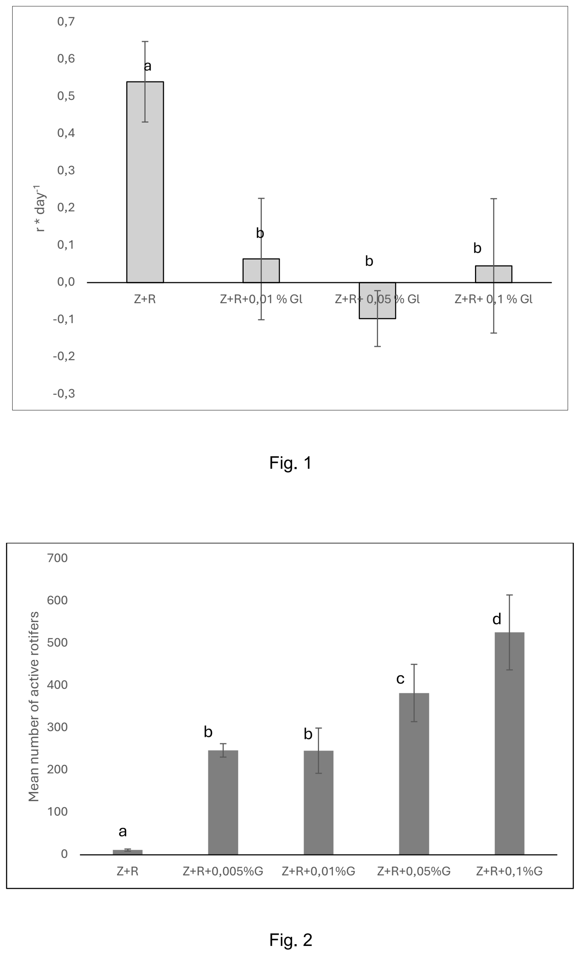

The highest growth rate was observed in the control group without glycerol supplementation and was approximately ten times higher than in the other groups. Figure 1 summarises the results of the study. Z+R denotes the control group (culture without glycerol); Z+R+GI (0.01%) denotes the group in which *L. inermis* and *Zoophagus sp.* Tk1 were cultured in the presence of glycerol at a concentration of 0.01%; Z+R+GI (0.05%) denotes the group in which the culture was maintained in the presence of glycerol at a concentration of 0.05%; Z+R+GI (0.1%) denotes the group in which the culture was maintained in the presence of glycerol at a concentration of 0.1%.

The growth rate of the fungus in all tested glycerol solutions was close to zero, and the differences between them were not significant (Fig. 1).

Studies were also conducted with mature mycelium. The experiments were carried out on two 12-well tissue culture plates. Three to five conidia were transferred to each well using a micropipette and left in small droplets to ensure that the spores adhered to the bottom. Rotifers were added *ad libitum.* After one day, 1 mL of Żywiec^{®} spring water and rotifers *ad libitum* were added to each well, and the plates were left for 10 days to allow the mycelium to grow. When the mycelium was well developed, the Żywiec^{®} water in each well was gently replaced with 2 mL of the appropriate solution to obtain five experimental groups: Z+R (control); Z+R+GI (0.005%); Z+R+GI (0.01%); Z+R+GI (0.05%); Z+R+GI (0.1%). Next, 58 µL of a dense culture containing approx. 800 rotifers was added to each well. After three consecutive days, all active rotifers were counted *in vivo.* The next day, an additional 400 rotifers in 20 µL of dense culture were added to each well, and a day later, the rotifers were preserved with Lugol's acid solution, and then all rotifers that had not been immobilised by the fungus were counted. The experiment was performed in 4 replicates per treatment. In the case of experiments with mature mycelium, just three days after replacing pure spring water (Zywiec) with a glycerol solution of varying concentrations and transferring approximately 800 *Lecane* individuals to each experimental well, only a few active rotifers remained in the control without glycerol addition.

As shown in Fig. 2, even at the lowest glycerol concentration (0.005%), the number of rotifers not captured by the fungus was approximately 50 times higher than in the control group. Similar results were observed for the 0.01% glycerol solution. Glycerol proved to be much more effective in protecting rotifers against the predatory fungus *Zoophagus* at higher concentrations: 0.05% and 0.1%, although no statistically significant difference in the number of active rotifers was found between these two treatments.

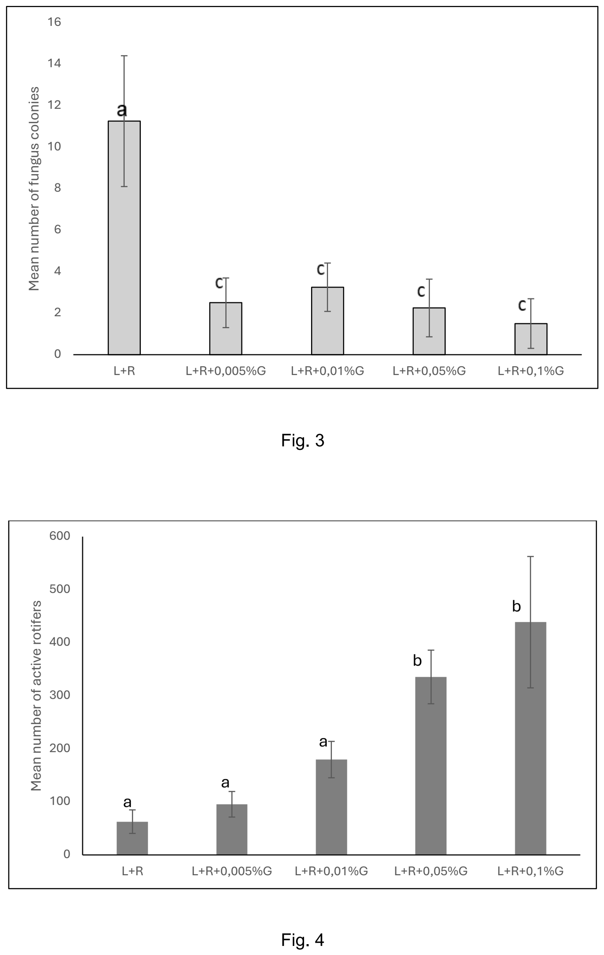

In the case of another fungus studied, i.e. *Lecophagus,* the effect of glycerol on its growth starting from conidia was also significant. A study was conducted involving the transfer of an equal volume of conidia suspension to wells in an experimental plate. Approximately 400 rotifers were added to each well. The harmful effect of the glycerol additive was reflected in the number of colonies counted on the 13^{th} day after replacing the standard medium with glycerol solutions of various concentrations. Approximately five times more fungal colonies were observed in the control without glycerol addition compared to all treatments with glycerol, with no significant differences between the treatments with glycerol (Fig. 3).

The protective effect of glycerol on rotifers was also apparent in the presence of *Lecophagus.* Even at a glycerol concentration as low as 0.01%, the number of active rotifers was approximately twice as high as in the control group, in which fungal predation was not limited by glycerol (Fig. 4). The number of rotifers that survived in the presence of the predatory fungus at the highest glycerol concentration was approximately eight times higher than in the control, while at a concentration of 0.01%, the number of rotifers not caught was on average three times higher than in the control.

## Claims

1. The use of glycerol to protect *Lecane* rotifers from the predation of fungi *Zoophagus* or *Lecophagus.*

2. The use of claim 1, **characterised in that** glycerol is used in a concentration ranging from 0.005% to 0.1%.

3. The use of claim 1, **characterised in that** *Lecane* rotifers are present in the activated sludge.

4. A method for controlling activated sludge bulking, comprising the use of rotifers, **characterised in that** the rotifers are introduced into the activated sludge in the presence of a glycerol solution at a concentration ranging from 0.005% to 0.1%.

5. The method of claim 4, **characterised in that** the activated sludge contains filamentous bacteria and is infected with strains of predatory fungi *Zoophagus* or *Lecophagus.*

6. The method of claim 4, **characterised in that** the rotifers are introduced into the activated sludge in the presence of a glycerol solution at a concentration ranging from 0.05% to 0.1%.
